# EUROPEAN PATENT APPLICATION

(11) **EP 2 537 546 A1**
(43) Date of publication of application: **26.12.2012**
(21) Application number: 11170828.5
(22) Date of filing: 21.06.2011
(51) Int. Cl.: A61M 5/145, A61M 5/168

(54) **Medicament delivery device with dose control mechanism**

(71) Applicant: Sanofi-Aventis Deutschland GmbH, 65929 Frankfurt (DE)
(72) Inventor: The designation of the inventor has not yet been filed

(57) **Abstract**

Described is a medicament delivery device (5) comprising a cartridge (15) containing a medicament, a pressure delivery mechanism (45) adapted to apply a pressure to the medicament, and a valve (55) disposed adjacent the cartridge (15). The valve (55) is adapted to regulate flow of the medicament from the cartridge as a function of the pressure.

## Description

### Background of the Invention

Patients suffering from diseases like diabetes and rheumatoid arthritis and patients restricted to bed-rest or in post-operative settings have to receive medicaments via injection (whether self-administered or from another individual, e.g., a physician). Medicament delivery devices, such as pen injectors, auto-injectors, pumps and syringes, have been developed to facilitate injections. The medicament delivery devices may be disposable single-dose or multi-dose devices or may be reusable and utilize replacement medicament cartridges.

In some conventional medicament delivery devices, manual force is utilized to deliver the medicament. For example, a patient is required to push a plunger of the syringe to deliver the medicament. For patients with limited dexterity or limited movement, such a task may be difficult. Additionally, in such cases, patients may have difficultly setting a dose or ensuring that an entire dose has been delivered. Thus, there is a need for a medicament delivery device with a dose control mechanism.

### Summary of the Invention

It is an object of the present invention to provide an medicament delivery device with a dose control mechanism.

In an exemplary embodiment, a medicament delivery device according to the present invention comprises a cartridge containing a medicament, a pressure delivery mechanism adapted to apply a pressure to the medicament, and a valve disposed adjacent the cartridge. The valve is adapted to regulate flow of the medicament from the cartridge as a function of the pressure.

The medicament may be one of insulin, complex carbohydrates, pharmaceutically active peptides, pharmaceutically active carbohydrates, pharmaceutically active nucleic acids, antibodies, a vaccine and a biological material.

In an exemplary embodiment, the pressure delivery mechanism is one or more of a spring, a pneumatic device and a motor. The device may further comprise an actuator which, upon actuation, causes the valve to be oriented in an open state. The actuator may be a button, a dial, a sensor or a switch. The valve may be one of a ball valve, a butterfly valve, a check valve, a diaphragm valve, a gate valve, a globe valve, a needle valve, a plug valve, a spool valve, and a piezoelectric element.

In an exemplary embodiment, the device further comprises a processor for controlling operation of the valve, and at least one visual indicator operatively coupled to the processor. The at least one visual indicator may include a first light-emitting-diode having a first color adapted to indicate the medicament delivery device is ready for use and a second light-emitting diode having a second color adapted to indicate the medicament delivery device is not ready for use.

In an exemplary embodiment, the device further comprises a plunger slidably disposed in the cartridge, and a first sensor generating a first signal based on movement of the plunger. The device may further comprise a dosing mechanism adapted to vary a dose of the medicament to be injected, and a second sensor generating a second signal based on use of the dosing mechanism. The processor may be adapted to trigger actuation of the valve between an open state and a closed state and to control operation of the pressure delivery mechanism based on the first signal and the second signal.

In an exemplary embodiment, the device further comprises a display displaying a dose amount, a power source providing power to the processor, a cap, and a cap sensor adapted to generate a cap sensor signal based on a presence of the cap.

In an exemplary embodiment, the processor is adapted to selectively illuminate at least one of the first and second light-emitting-diodes as a function of the cap sensor signal. The processor may also be adapted to selectively enable the pressure delivery mechanism as a function of the cap sensor signal.

In an exemplary embodiment, the medicament is pressurized within the cartridge.

Further scope of applicability of the present invention will become apparent from the detailed description given hereinafter. However, it should be understood that the detailed description and specific examples, while indicating preferred embodiments of the invention, are given by way of illustration, since various changes and modifications within the spirit and scope of the invention will become apparent to those skilled in the art from this detailed description.

### Brief Description of the Drawings

Figure 1 shows an exemplary embodiment of an medicament delivery device according to the present invention; and
Figure 2 shows an exemplary embodiment of a medicament delivery device according to the present invention.

Corresponding parts are marked with the same reference symbols in all figures.

### Detailed Description

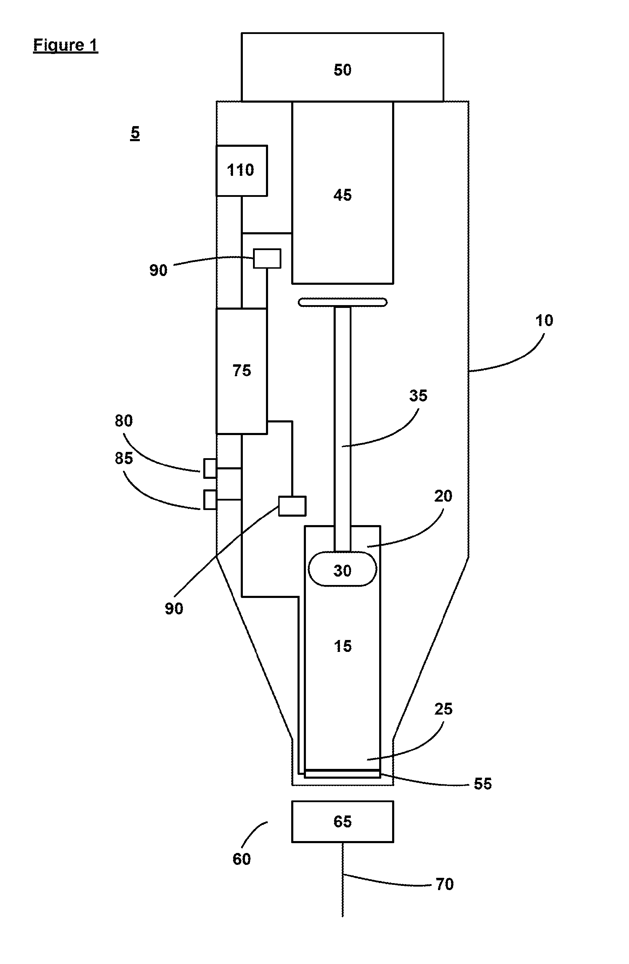

Figure 1 shows an exemplary embodiment of a medicament delivery device 5 according to the present invention. In the exemplary embodiment shown in Figure 1, the medicament delivery device 5 is depicted as a pen injector or an auto-injector. However, those of skill in the art will understand that the medicament delivery device 5 according to the present invention may include pen injectors, auto-injectors, pumps, syringes and other transdermal fluid delivery systems. For example, the medicament delivery device 5 may be utilized to deliver medicaments or other therapeutic agents into an intravenous port or a perfusion system.

In the exemplary embodiment shown in Figure 1, the medicament delivery device 5 comprises a housing 10 which may have an elongate shape to facilitate usability, e.g., for a patient with limited dexterity. Thus, the housing 10 may be elongate and ergonomically shaped (e.g., like a bicycle handlebar) to assist with gripping and holding the medicament delivery device 5.

Disposed within the housing 10 may be a cartridge 15 containing a medicament. In an exemplary embodiment, the housing 10 may be formed from a single component (e.g., in which the medicament delivery device 5 is disposable) or from multiple components (e.g., in which the medicament delivery device 5 is reusable and a used or empty cartridge may be replaced with a new, full cartridge).

In an exemplary embodiment, the cartridge 15 includes a proximal portion 20 and a distal portion 25. A bung 30 may be disposed in the proximal portion 20 of the cartridge 15 and be axially movable with the cartridge 15. As the bung 30 moves from the proximal portion 20 to the distal portion 25, the medicament in the cartridge 15 is dispensed. Extending axially and proximally from the bung 30 is a plunger 35. In an exemplary embodiment, the plunger 35 is formed integrally with the bung 30. In another exemplary embodiment, the plunger 35 is separate from the bung 30, but is adapted to engage the bung 30 during delivery of the medicament. A proximal end of the plunger 35 may include an abutment face 40. The abutment face 40 may be formed as, for example, a disc or other shape to receive a distally directed pressure for displacing the plunger 35.

Disposed proximally of the plunger 35 is a pressure delivery mechanism 45 which is adapted to, when activated, apply pressure to the proximal end of the plunger 35. In an exemplary embodiment, the pressure delivery mechanism 45 is a pre-loaded spring. A distal end of the spring may abut the abutment face 40 of the plunger 35, and a proximal end of the spring may abut an inner surface of the housing 10 or a structure formed therein for preventing proximal movement of the spring. In another exemplary embodiment, the pressure delivery mechanism 45 may be a pneumatic device (e.g., a carbon dioxide cartridge) which, when activated, forces a piston in a distal direction to abut the abutment face 40 of the plunger 35. In another exemplary embodiment, the pressure delivery mechanism 45 may be a motor which, when activated, drives a piston in a distal direction to abut the abutment face 40 of the plunger 35. Those of skill in the art will understand that various other mechanisms for generating positive pressure may be utilized. The pressure delivery mechanism 45 may be capable of generating negative pressure to, for example, draw the plunger 35 in a proximal direction when resetting a reusable medicament delivery device.

An actuator 50 may be disposed on the medicament delivery device 5. In an exemplary embodiment, the actuator 50 may be a button disposed at a proximal end of the medicament delivery device 5. When the patient is orienting the medicament delivery device 5 for use, the button may be arranged for thumb-activation. Those of skill in the art will understand that other types of actuators (e.g., a switch, a dial, a sensor, etc.) may be utilized. Pressing (or otherwise actuating) the actuator 50 initiates delivery of the medicament, as explained further below.

In an exemplary embodiment, a valve 55 is disposed at the distal portion 25 of the cartridge 15. The valve 55 is adapted to regulate flow of the medicament from the cartridge 15. In an exemplary embodiment, the valve 55 is biased in a closed state. The valve 55 may be any type of valve which includes, but is not limited to a ball valve, a butterfly valve, a check valve, a diaphragm valve, a gate valve, a globe valve, a needle valve, a plug valve, a spool valve, etc. In an exemplary embodiment, the valve 55 may include a piezoelectric element which changes shape to regulate flow of the medicament from the cartridge 15. In an exemplary embodiment, the valve 55 may be disposed adjacent to a septum formed at the distal portion 25 of the cartridge 15.

A needle assembly 60 may be removably coupled to the distal end of the housing 10 or to the distal portion 25 of the cartridge 15. The needle assembly 60 may include a needle hub 65 for engaging the distal end of the housing 10 or the distal portion 25 of the cartridge 15, e.g., via a threaded, bayonet, or frictional engagement. A needle 70 may project distally from the needle hub 65 for delivering the medicament to the patient. In an exemplary embodiment, the needle 70 may be a double-sided needle with a distal tip for piercing the patient's skin and a proximal tip for piercing a septum on the cartridge 15.

In an exemplary embodiment, the medicament delivery device 5 may include a processor 75. The processor 75 may include memory for storing instructions to operate components of the medicament delivery device 5. The processor 75 may be operatively coupled to the valve 55 to control, for example, a rate at which the valve 55 transitions from the closed state to an open state.

The processor 75 may also be coupled to one or more visual indicators (e.g., LEDs). For example, the medicament delivery device 5 may include a first LED 80 (e.g., green LED) which, when illuminated, indicates that the medicament delivery device 5 is ready to deliver a dose of the medicament, and a second LED 85 (e.g., a red LED) which, when illuminated, indicates that the medicament delivery device 5 is not ready to deliver a dose of the medicament. Different illumination patterns may also be used. For example, illumination of the first and second LEDs 80, 85 simultaneously or alternately may indicate that the medicament delivery device 5 has encountered an error and requires repair or replacement. Illumination of the second LED 85 in a blinking fashion may indicate that the cartridge 15 is empty or that a power source in the medicament delivery device 5 needs to be replaced. Those of skill in the art will understand that various illumination patterns, colors and/or brightnesses may be utilized with the one or more visual indicators to provide information about the medicament delivery device 5 to the patient. Further, those of skill in the art will understand that an audible indicator(s) may be utilized in place of or with the visual indicators. Different volumes, frequencies and/or alarm patterns may be utilized to provide information about the medicament delivery device 5 to the patient.

In an exemplary embodiment, the processor 75 may be operatively coupled to a first sensor 90 which senses motion of the plunger 35. The processor 75 may utilize signals returned from the first sensor 90 to determine an amount of medicament remaining in the cartridge 15. The first sensor 90 may be for example, an optical sensor which receives a reflected signal from a (textured) surface of the plunger 35. In another exemplary embodiment, the first sensor 90 may be a mechanical sensor having a movable lever or gear which mates with a rack on the plunger 35.

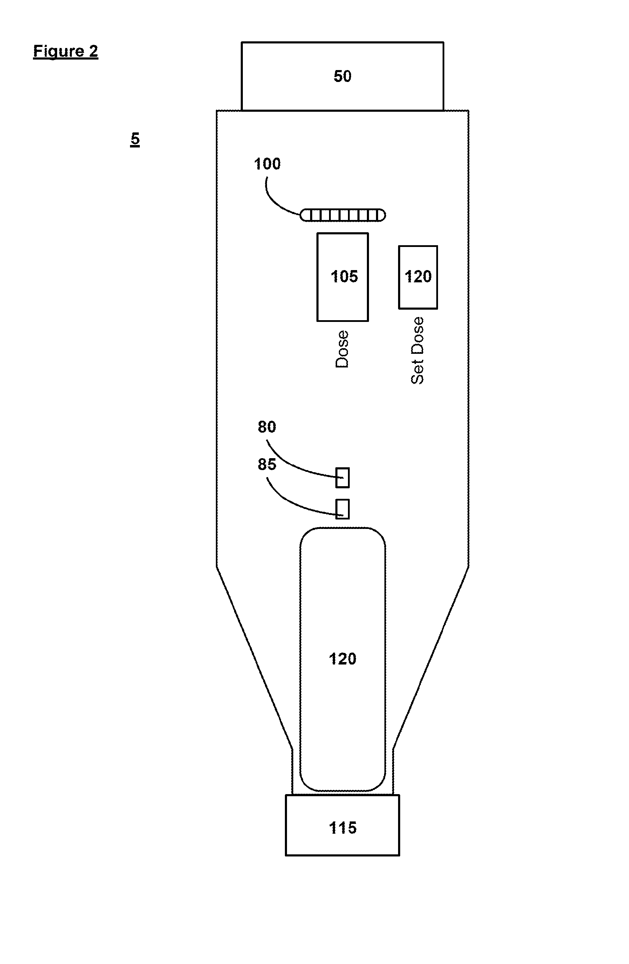

In an exemplary embodiment, the processor 75 is coupled to a second senor 95 which senses adjustment of a dosing mechanism 100 (shown in Figure 2). The processor 75 may utilize signals returned from the second sensor 95 to update information shown on a display 105. For example, the information shown in the display 105 may indicate a dose amount, an amount of medicament remaining in the cartridge 15, a date by which the medicament should be used, device operating instructions, etc.

In an exemplary embodiment, the processor 75 is also coupled to a power source 110 (e.g., a disposable battery, a rechargeable battery, etc.) and the pressure delivery mechanism 45. If the power source 110 is rechargeable, the medicament delivery device 5 may include a port for connecting an external power to the power source 110 for re-charging.

In an exemplary embodiment, the medicament delivery device 5 may include a needle shield disposed at a distal end. The needle shield may have an extended position and a retracted position. Prior to use the needle shield may be in the extended position. When the medicament delivery device 5 is placed on the injection site, the needle shield may move into the retracted position and expose the needle 70. After use the needle shield may lock in the extended position. Preferably, the needle shield is biased in the extended position. If the medicament delivery device 5 is reusable, the needle shield may be unlocked (e.g., manually or by the processor 75, e.g., after setting a new dose) to deliver a subsequent dose of the medicament.

Figure 2 shows an exemplary embodiment of the medicament delivery device 5 according to the present invention. A distal opening of the medicament delivery device 5 may include a cap 115. The cap 115 may be coupled to the medicament delivery device 5 by, for example, a threaded engagement, a bayonet fit, a frictional fit, etc., and the cap 115 may be disposable (e.g., discarded after removal) or replaceable (e.g., re-coupleable to the medicament delivery device 5 between uses). A cap sensor may be utilized to detect a presence of the cap 115. The processor 75 may utilize signals received from the cap sensor to prevent inadvertent use of the medicament delivery device 5.

A window 120 may be formed in the housing 10 to allow the patient to have visual access to the medicament.

Prior to use, the medicament delivery device 5 may be embodied as the exemplary embodiment shown in Figure 2. The patient may set a dose of the medicament to be delivered using the dosing mechanism 100. The dosing mechanism 100 may be a wheel, one or more switches, one or more push buttons, etc. which the patient can use to increase and/or decrease the dose amount. As the patient interacts with the dosing mechanism, the dose amount is updated on the display 105.

When the patient has entered the desired dose amount, the patient may press a button 125 disposed on the medicament delivery device 5 to enter the dose amount. When the patient has pressed the button 125 to enter the dose amount, the processor 75 may receive a signal from the button 125 and, in response, disable the dosing mechanism 100.

After the dose amount has been entered, the patient may remove the cap 115. When the cap 115 is removed from the medicament delivery device 5, the processor 75 may receive a signal from the cap sensor. In response to the signal from the cap sensor, the processor 75 may illuminate the second LED 85 to notify the patient that the medicament delivery device 5 is not yet ready for use.

In an exemplary embodiment, when the processor 75 receives the signal from the cap sensor, the processor 75 may activate the pressure delivery mechanism 45 to advance the plunger 35 distally, thereby applying pressure to the medicament in the cartridge 15. However, the processor 75 may maintain the valve 55 in the closed state, which creates a pressure differential proximal of the valve 55. When the processor 75 has caused the pressure delivery mechanism 45 to apply the proper pressure to the medicament (e.g., based on the entered dose amount and the amount of medicament remaining in the cartridge 15), the processor may illuminate the first LED 80 to notify the patient that the medicament delivery device 5 is ready for use. When the medicament delivery device 5 has been placed on an injection site, the patient may actuate the actuator 50. The processor 75 may sense that the actuator 50 has been actuated and open the valve 55 to allow the medicament to flow from the cartridge 15, through the needle 70 and into the injection site. After the dose amount has been delivered, the processor 75 may close the valve 55 and illuminate the second LED 85 to notify the patient that the dose amount has been delivered and the medicament delivery device 5 is no longer ready for use. After delivering an injection, the patient may reattach the cap 115 to the medicament delivery device 5.

In another exemplary embodiment, when the processor 75 receives the signal from the cap sensor, the processor may illuminate the first LED 80 to notify the patient that the medicament delivery device 5 is ready for use. When the medicament delivery device 5 has been placed on an injection site, the patient may actuate the actuator 50. The processor 75 may sense that the actuator 50 has been actuated and may activate the pressure delivery mechanism 45 to advance the plunger 35 distally, thereby applying pressure to the medicament in the cartridge 15. When the processor 75 has caused the pressure delivery mechanism 45 to apply the proper pressure to the medicament (e.g., based on the entered dose amount and the amount of medicament remaining in the cartridge 15), the processor 75 may open the valve 55 to allow the medicament to flow from the cartridge 15, through the needle 70 and into the injection site. After the dose amount has been delivered, the processor 75 may close the valve 55 and illuminate the second LED 85 to notify the patient that the dose amount has been delivered and the medicament delivery device 5 is no longer ready for use. After delivering an injection, the patient may reattach the cap 115 to the medicament delivery device 5.

In another exemplary embodiment, the medicament delivery device 5 may include a receiver for receiving wireless communication signals or a transceiver for transmitting and receiving wireless communications signals. The wireless communications signals may be transmitted/received according to any wireless communication protocol (e.g., 802.11x, xDMA, OFDM, Bluetooth®, etc.). In this exemplary embodiment, operation of the medicament delivery device 5 may be controlled and/or monitored remotely. For example, the dose amount may be set remotely and/or a signal indicating that the cartridge 15 is empty may be sent from the medicament delivery device 5 to a remote monitoring device (e.g., a mobile phone, a PDA, a blood glucose meter, etc.).

The medicament which may be delivered by the medicament delivery device 5 according to the present invention includes, but is not limited to, a pharmaceutical formulation containing at least one pharmaceutically active compound,
wherein in one exemplary embodiment the pharmaceutically active compound has a molecular weight up to 1500 Da and/or is a peptide, a protein, a polysaccharide, a vaccine, a DNA, a RNA, , an enzyme, an antibody or a fragment thereof, a hormone or an oligonucleotide, or a mixture of the above-mentioned pharmaceutically active compound,
wherein in a further exemplary embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further exemplary embodiment the pharmaceutically active compound comprises at least one peptide for the treatment and/or prophylaxis of diabetes mellitus or complications associated with diabetes mellitus such as diabetic retinopathy,
wherein in a further exemplary embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, lsoAsp28] Exendin-4(1-39),
des Pro36 [Trp(02)25, Asp28] Exendin-4(1-39),
des Pro36 [Trp(02)25, lsoAsp28] Exendin-4(1-39),
des Pro36 [Met(O)14 Trp(02)25, Asp28] Exendin-4(1-39),
des Pro36 [Met(O)14 Trp(02)25, lsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, lsoAsp28] Exendin-4(1-39),
des Pro36 [Trp(02)25, Asp28] Exendin-4(1-39),
des Pro36 [Trp(02)25, lsoAsp28] Exendin-4(1-39),
des Pro36 [Met(O)14 Trp(02)25, Asp28] Exendin-4(1-39),
des Pro36 [Met(O)14 Trp(02)25, lsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and K. A light chain has two successive domains: one constant domain (CL) and one variable domain (VL). The approximate length of a light chain is 211 to 217 amino acids. Each antibody contains two light chains that are always identical; only one type of light chain, K or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand the modifications (additions and/or removals) of various components of the device and/or system and embodiment described herein may be made without departing from the full scope and spirit of the present invention, which encompass such modifications and any and all equivalents thereof.

### List of References

- 5: medicament delivery device
- 10: housing
- 15: cartridge
- 20: proximal portion
- 25: distal portion
- 30: bung
- 35: plunger
- 40: abutment face
- 45: pressure delivery mechanism
- 50: actuator
- 55: valve
- 60: needle assembly
- 65: needle hub
- 70: needle
- 75: processor
- 80: first LED
- 85: second LED
- 90: first sensor
- 95: second sensor
- 100: dosing mechanism
- 105: display
- 110: power source
- 115: cap
- 120: window
- 125: button

## Claims

1. A medicament delivery device (5) comprising:
a cartridge (15) containing a medicament;
a pressure delivery mechanism (45) adapted to apply a pressure to the medicament; and
a valve (55) disposed adjacent the cartridge (15),
wherein the valve (55) is adapted to regulate flow of the medicament from the cartridge as a function of the pressure.

2. The medicament delivery device (5) according to claim 1, wherein the medicament is one of insulin, complex carbohydrates, pharmaceutically active peptides, pharmaceutically active carbohydrates, pharmaceutically active nucleic acids, antibodies, a vaccine and a biological material.

3. The medicament delivery device (5) according to anyone of the preceding claims, wherein the pressure delivery mechanism (45) is one or more of a spring, a pneumatic device and a motor.

4. The medicament delivery device (5) according to anyone of the preceding claims, further comprising:
an actuator (50) which, upon actuation, causes the valve (55) to be oriented in an open state.

5. The medicament delivery device (5) according to claim 4, wherein the actuator (50) is one of a button, a dial, a sensor and a switch.

6. The medicament delivery device (5) according to anyone of the preceding claims, wherein the valve (55) is one of a ball valve, a butterfly valve, a check valve, a diaphragm valve, a gate valve, a globe valve, a needle valve, a plug valve, a spool valve, and a piezoelectric element.

7. The medicament delivery device (5) according to anyone of the preceding claims, further comprising:
a processor (75) controlling operation of the valve (55).

8. The medicament delivery device (5) according to claim 7, further comprising:
at least one visual indicator (80, 85) operatively coupled to the processor (75).

9. The medicament delivery device (5) according to claim 8, wherein the at least one visual indicator includes a first light-emitting-diode (80) having a first color adapted to indicate the medicament delivery device (5) is ready for use and a second light-emitting diode (85) having a second color adapted to indicate the medicament delivery device (5) is not ready for use.

10. The medicament delivery device (5) according to claim 7, further comprising:
a plunger (35) slidably disposed in the cartridge (15); and
a first sensor (90) generating a first signal based on movement of the plunger (35).

11. The medicament delivery device (5) according to anyone of the preceding claims, further comprising:
a dosing mechanism (100) adapted to vary a dose of the medicament to be injected; and
a second sensor (95) generating a second signal based on use of the dosing mechanism (100).

12. The medicament delivery device (5) according to claims 10 and 11, wherein the processor (75) is adapted to trigger actuation of the valve (55) between an open state and a closed state and to control operation of the pressure delivery mechanism (45) based on the first signal and the second signal.

13. The medicament delivery device (5) according to anyone of the preceding claims, further comprising:
a display (105) displaying a dose amount.

14. The medicament delivery device (5) according to claim 7, further comprising:
a power source (110) providing power to the processor (75).

15. The medicament delivery device (5) according to claim 9, further comprising:
a cap (115); and
a cap sensor adapted to generate a cap sensor signal based on a presence of the cap (115).

16. The medicament delivery device (5) according to claim 15, wherein the processor (15) is adapted to selectively illuminate at least one of the first and second light-emitting-diodes (80, 85) as a function of the cap sensor signal.

17. The medicament delivery device (5) according to claim 15, wherein the processor (15) is adapted to selectively enable the pressure delivery mechanism (45) as a function of the cap sensor signal.

18. The medicament delivery device (5) according to any one of the preceding claims, wherein the medicament is pressurized within the cartridge (15).
